# EUROPEAN PATENT APPLICATION

(11) **EP 1 256 350 A1**
(43) Date of publication of application: **13.11.2002**
(21) Application number: 01905602.7
(22) Date of filing: 13.02.2001
(51) Int. Cl.: A61K 35/78, A61K 31/21

(54) **NEW TORALACTONE AND ITS DERIVATION AND THE USE OF DECREASING BLOOD-FAT AND LOSING WEIGHT**

(30) Priority: 16.02.2000 CN 00100895
(71) Applicant: Pan, Xiaodong, Beijing 100039 (CN)
(72) Inventor: Pan, Xiaodong, Beijing 100039 (CN)
(74) Representative: Harding, Charles Thomas
(86) International application number: CN0100123
(87) International publication number: WO01060390

(57) **Abstract**

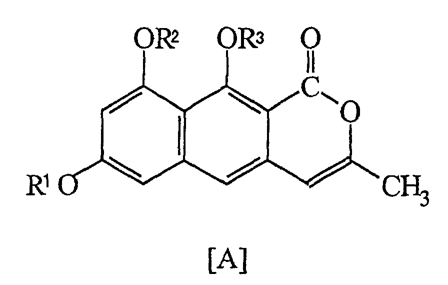

A class of novel toralactones of general formula [A] extracted from *Semen Cassiae*, wherein R³ is H or C₂-C₄ acyl, R¹, R² are same or different, and are each H, glycosyl, or C₂-C₄ acyl, and the derivatives thereof. Said compounds can decrease the level of total serum cholesterol and increase the level of bile acid, manifesting that these compounds have the efficacy of reducing blood fat and reducing weight.

## Description

### Field of the Invention

The present invention relates to a class of novel compounds extracted from *Semen Cassiae*, and their use in reducing blood fat and reducing weight.

### Background of the Invention

*Semen Cassiae* are dried mature seeds of a leguminous plant, *Cassia obtusifolia* L., which has the effect of dispelling pathogenic wind and heat, removing heat from the liver and improving eyesight, and loosening the bowel to relieve constipation. *Semen Cassiae* is used to decrease the level of serum cholesterol in clinical practice. *Semen Cassiae* contains a variety of anthraquinone and benzopyrone components. It has been reported that among those components, some anthraquinone glycosides and benzopyrone glycosides have the efficacy of preventing the damage of tetrachloromethane and galactosamine to the primary hepatic cell culture of mouse (Qiwei, Zhang, et al., *Chinese Traditional and Herbal Medicine,* 1996, 27 (2): 79; Wong, SM, et al., *Planta Med*. 1989, 55 (3): 276 and 28 (1): 211).

Many medicaments for reducing blood fat and reducing weight, prepared from *Semen Cassiae* and other traditional Chinese medicine, have been proposed in many patent applications. However, their complicated composition makes it difficult to identify the active ingredients that play the key role.

Takahashi et al. reported their results from studies on the components of the seeds of *Cassia tora* L. in Yakugaku Zasshi 93 (3) 261-267 (1973), in which the compounds of the following general formula were disclosed: 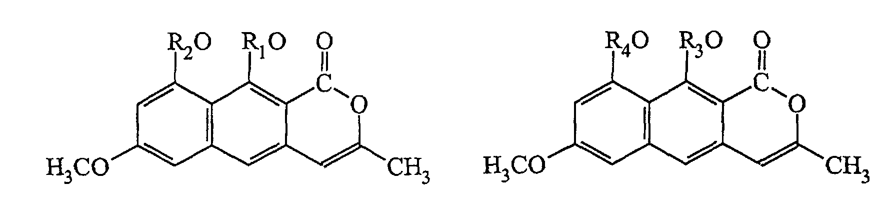
I: R₁, R₂=H
II: R₁, R₂=COCH₃
III: R₁, R₂=CH₃
IV: R₁=H, R₂=COCH₃
VI: R₁=CH₃, R₂=H
V: R₃=H, R₄=COCH₃
wherein Compound I, named as toralactone, is a yellow dye. However, the pharmaceutical effect of the compound was not reported therein.

The object of the present invention is to provide a class of novel toralactone compounds, which has the efficacy of reducing blood fat and reducing weight.

### Summary of the Invention

The present invention provides a class of novel toralactones of general formula [A] and derivatives thereof, which are extracted from *Semen Cassiae*, or obtained by modifying the structure of the extracted compounds: 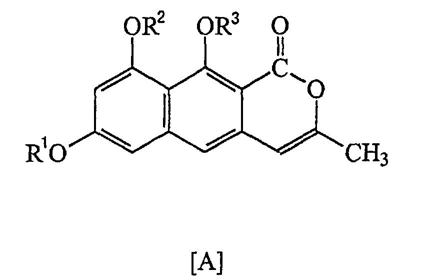 wherein R³ is H or C₂-C₄ acyl, R¹, R² are same or different, and are each H, glycosyl or C₂-C₄ acyl.

The present invention further provides the use of the above compounds in reducing blood fat and reducing weight, and the pharmaceutical compositions comprising such compounds.

### Detailed Description of the Invention

The present inventor has taken a long-term study on the use of the extracts from *Semen Cassiae* in reducing blood fat. The extraction of *Semen Cassiae* comprises the following steps:
1. Pulverizing *Semen Cassiae* (produced in Anhui Province, China), extracting with hydrophilic solvent, such as water, methanol, ethanol, acetone, or the like, and heating to reflux, then removing the solvent under reduced pressure to yield Extract (A);
2. Loading Extract (A) on a porous resin, washing with water, and eluting with hydrophilic solvent, such as methanol, ethanol, acetone or the like, then collecting the eluent and removing the solvent under reduced pressure to yield Extract (B);
3. Performing column chromatography on Extract (B) using Silica Gel H, Silica Gel G or aluminum hydroxide and eluting with chloroform, ethyl acetate, ethanol or methanol, then combining the fractions that have the same components as detected by TLC, and removing the solvent, recrystallizing with methanol to yield Compound (1), Compound (2), Compound (3), Compound (4) and Compound (5):

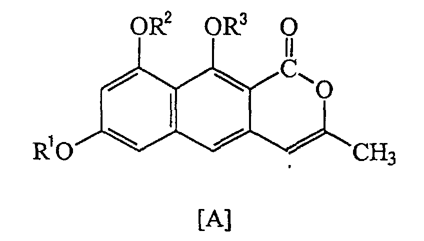

| Compound | R¹ | R² | R³ |
|---|---|---|---|
| (1) | H | H | H |
| (2) | glucosyl | H | H |
| (3) | H | Glucosyl | H |
| (4) | galactosyl | H | H |
| (5) | CH₃ | H | H |

wherein Compound (5) is the known toralactone, i.e. the above-mentioned compound I. Compounds (1) - (4) are novel compounds, which are named as:
(1) neotoralactone, or nortoralactone;
(2) neotoralactone-6-O-β-D-glucoside
(3) neotoralactone-8-O-β-D-glucoside
(4) neotoralactone-6-O-β-D-galactoside

Further synthetic studies have shown that R¹, R², R³ can be further acylated to obtain compounds wherein R¹, R² and R³ are independently C₁-C₄ acyl, e.g. Compound (6):

| Compound | R¹ | R² | R³ |
|---|---|---|---|
| (6) | COCH₃ | COCH₃ | COCH₃ |

Instrumental analysis has confirmed that the extracted Compounds (1) - (5) have the above-mentioned structures.

Experiments on animals with these compounds have shown that, Compounds (1) - (4) have the effect of modulating serum cholesterol in hyperlipemia guinea pigs, and they contribute to the prevention of obesity. They have the efficacy of reducing blood fat and reducing weight.

The present invention will be further illustrated by the following examples and test examples, which are not intended to limit the scope of the invention.

### Example 1

### Extraction and structure characterization of Compounds (1) - (5)

Compounds (1) - (5) were extracted by the following steps:
1. Pulverizing *Semen Cassiae* (seeds of *Cassia obtusifolia* and *Cassia tora* (produced in Anhui Province, China) into coarse powders, extracting with 10 L of ethanol, while heating to reflux, then removing the solvent under reduced pressure to yield 210 g of Extract (A);
2. Loading 210 g of Extract (A) on 1000 g of a porous resin obtained by mixing Porous Resin D101, D201, D061 (produced by Tianjin Agriculture Pesticide Factory) in a ration of 1:1:1, then washing with 10 L of water and 5 L of ethanol sequentially, collecting the eluent, and removing the solvent to yield 10 g of Extract (B);
3. Performing column chromatography on 10 g of Extract (B) using 1000 g of Silica Gel H and eluting with chloroform, combining the portions that had the same constituents as detected by TLC, then removing the solvent, recrystallizing with methanol to yield Compound (1) (0.02 g), Compound (2) (2.42 g), Compound (3) (1.05 g), Compound (4) (0.5 g) and Compound (5) (0.32 g).

Compound (1) was in the form of yellow acicular crystal, with m.p. 187-189°C(methanol). Its molecular weight determined by high resolution mass spectrum was 258.052849, and the molecular formula was C₁₄H₁₀O₅, EI-MS (m/e): 258, 229, 162, 115, 69. The data from ¹³C-NMR and ¹H-NMR is shown in Table 1 and Table 2. Compound (1) was identified as neotoralactone (i.e. nortoralactone).

Compound (2) was in the form of yellow acicular crystal, with m.p. 252-254°C(methanol). FAB-MS (m/e): 421 (M+1), 259 (aglucone+1), 235. The data from ¹³C-NMR and ¹H-NMR is shown in Table 1 and Table 2. Compound (2) gave Compound (1) and glucose after hydrolyzed in the presence of diluted acid. ¹³C-NMR indicated that glycosyl was bonded to the hydroxy at position 6. Compound (2) was identified as neotoralactone-6-O-β-D-glucoside.

Compound (3) was in the form of yellow acicular crystal, with m.p. 263-265°C(methanol). FAB-MS (m/e): 421 (M+1), 259 (aglucone+1), 235. The data from ¹³C-NMR and ¹H-NMR is shown in Table 1 and Table 2. Compound (3) gave Compound (1) and glucose after hydrolysis. 13C-NMR indicated that glycosyl was bonded to the hydroxy at position 8. Compound (3) was identified as neotoralactone-8-O-β-D-glucoside.

Compound (4) was in the form of yellow acicular crystal, with m.p. 258-260°C(methanol). FAB-MS (m/e): 421, 259 (aglucone+1), 235. The data from ¹³C-NMR and ¹H-NMR is shown in Table 1 and Table 2. Compound (4) gave Compound (1) and galactose after hydrolysis. ¹³C-NMR indicated that glycosyl was bonded to the hydroxy at position 6.

Compound (4) was identified as neotoralactone-6-O-β-D-galactoside.

Compound (5) was in the form of yellow acicular crystal, with m.p. 234-236 °C (methanol). R_{f} value from TCL was the same as that of toralactone.

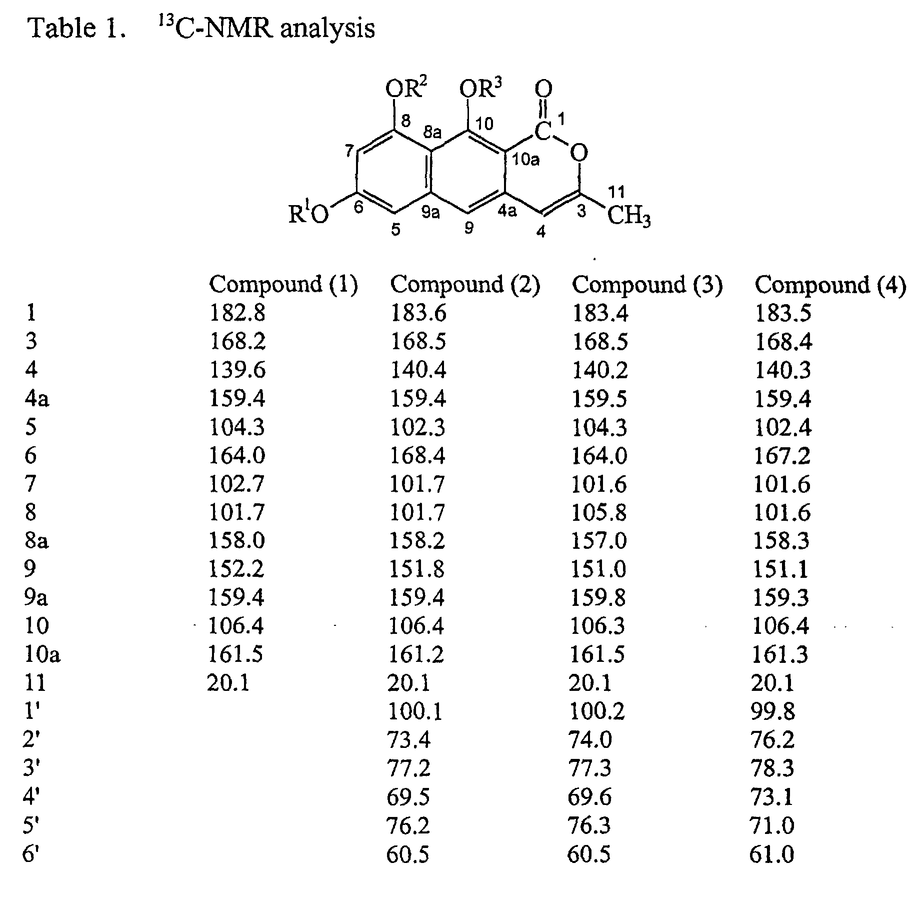

**Table 2.**

| ¹H-NMR analysis | | | | |
|---|---|---|---|---|
| | Compound (1) | Compound (2) | Compound (3) | Compound (4) |
| 4 | 6.6(s) | 6.6(s) | 6.6(s) | 6.6(s) |
| 5 | 7.0(d.s=3.5) | 7.0(d) | 7.1(d) | 7.1(d) |
| 6 | 10.2(s) | -- | 10.2(s) | -- |
| 7 | 5.9(d.s=3.5) | 6.1(d) | 6.0(d) | 6.0(d) |
| 8 | 10.4(s) | 10.3(s) | -- | 10.3(s) |
| 9 | 15.0(s) | 14.9(s) | 14.9(s) | 14.9(s) |
| 10 | 6.7(s) | 6.7(s) | 6.7(s) | 6.7(s) |
| 11 | 2.5(s) | 2.5(s) | 2.5(s) | 2.5(s) |
| 1¹ | | 4.9(d.J=7.6) | 5.2(d.J=7.6) | 4.7(d.J=7.6) |
| 2¹-6¹ | | 2.9-3.8(m) | 2.9-3.8(m) | 2.9-3.8(m) |

### Example 2

### Preparation of Compound (6)

10 mg of Compound (1) was dissolved in pyridine, to which 2 ml of acetic anhydride was added. After standing for 24 hours, the mixture was poured into ice water. After filtration, the acetylated Compound (1), i.e. Compound (6) was yielded.

Compound (6) was in the form of yellow acicular crystal, with m.p. 156-158°C. FAB-MS (m/e): 385 (M⁺+1), 384 (M⁺), 342 (M⁺-COCH₃), 299 (M⁺-2×COCH₃), 257 (M⁺-3×COCH₃), 162, 115. Compound (6) was identified as 6,8,10-triacetylneotoralactone.

### Test Example 1

### The effect of neoalactone and its derivatives on the level of serum cholesterol in hyperlipemia guinea pigs

The test was performed as described in Fuhua, Wang, *Chinese Journal of Surgery*, 1991; 8 (1): 17. 60 healthy guinea pigs of 200-500 g were randomly divided into 6 groups based on body weight and sex. Each group contained 10 animals, including both male and female. The guinea pigs of Group 1 were fed with high-cholesterol feed (basic feed + 0.5% cholesterol) at an amount of 20 g per 100g body weight per day. Groups 2, 3, 4, 5 were test groups, the guinea pigs of which were fed with high-cholesterol feed plus 10 mg/(kg·d) of Compound (1), (2), (3) or (4) for 30 days. The guinea pigs of Group 6 were fed with basic feed only. The feeding for each group continued for 30 days, and 24 hours after the last feeding, the guinea pigs were anaesthetized i.p. with 1 g/kg Urethane. Blood was taken from heart, and serum was separated. The level of serum total cholesterol (TC) and triglyceride (TG) were determined. The results were shown in Table 3.

**Table 3.**

| The effect of the active compounds on the level of serum cholesterol and triglyceride in hyperlipemia guinea pigs | | | | |
|---|---|---|---|---|
| Group | Numbers of guinea pigs | Dosage mg/kg | Serum | |
| | | | TC (mmol/L) | TG (mmol/L) |
| Normal | 10 | --- | 3.75±1.5 | 1.28±0.2 |
| High cholesterol | 10 | | 4.80±1.4 | 1.87±0.8 |
| Compound (1) | 10 | 10 | 4.21±1.9 | 1.21±0.3 |
| Compound (2) | 10 | 10 | 2.68*±0.2 | 0.78**±0.2 |
| Compound (3) | 10 | 10 | 3.81±0.9 | 1.18±0.2 |
| Compound (4) | 10 | 10 | 3.71±1.1 | 1.22±0.2 |
| Compared with Normal: *P<0.05, **P<0.01 | | | | |

It can be seen from Table 3 that, the level of total serum cholesterol increased whereas the level of bile acid decreased when 0.05% of cholesterol was incorporated into the basic feed. The level of serum TC and TG could be reduced when the active compounds were fed at an amount of 10 mg/(kg·d)×30d. And the effect of Compound (2) was most prominent. This indicates that these compounds have the effect of modulating blood fat and promoting the balance of lipid metabolism. They contribute to the prevention of hyperlipemia obesity. And Compound (2) is preferred.

In the above procedure, cholesterol was determinated by enzymatic analysis, using enzymatic agent assay kit from BGH Company (Taiwan). All the operations were carried out according to the manufacturer's instruction, and the determination was accomplished in ENCOREII automatic biochemistry analyser.

## Claims

1. A compound of general formula [A]: 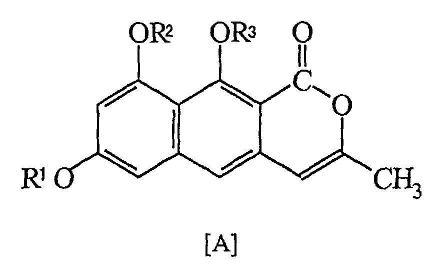 wherein R³ is H or C₂-C₄ acyl, R¹, R² are same or different, and are each H, glycosyl or C₂-C₄ acyl.

2. The compound of claim 1, wherein R³ is H, R¹ is H, and R² is H.

3. The compound of claim 1, wherein R³ is H, R¹ is glucosyl, and R² is H.

4. The compound of claim 1, wherein R³ is H, R¹ is H, and R² is glucosyl.

5. The compound of claim 1, wherein R³ is H, R¹ is galactosyl, and R² is H.

6. The compound of claim 1, wherein R¹, R² and R³ are acetyl.

7. Use of the compound of any one of the preceding claims 1-6 in the preparation of medicaments for reducing blood fat and reducing weight.

8. A pharmaceutical composition comprising the compound of claim 1, and pharmaceutically acceptable carriers.

9. The pharmaceutical composition of claim 8, wherein said compound is neotoralactone-6-O-β-D-glucoside.
